# EUROPEAN PATENT SPECIFICATION

(11) **EP 1 128 809 B1**
(45) Date of publication and mention of the grant of the patent: **29.12.2004**
(21) Application number: 98954658.5
(22) Date of filing: 20.11.1998
(51) Int. Cl.: A61K 9/00, A61K 47/36

(54) **AQUEOUS OPHTHALMIC FORMULATIONS COMPRISING CHITOSAN**
WÄSSRIGE OPHTHALMISCHE FORMULIERUNGEN MIT CHITOSAN
FORMULATIONS OPHTALMIQUES AQUEUSES COMPRENANT DU CHITOSANE

(43) Date of publication of application: 05.09.2001
(73) Proprietor: LABORATOIRE MEDIDOM S.A., 1206 Geneva (CH)
(72) Inventor: GURNY, Robert, CH-1204 Geneva (CH); FELT, Olivia, F-74160 Collonges sous Salève (FR)
(74) Representative: Reuteler, Raymond Werner
(86) International application number: PCT/IB1998/001886
(87) International publication number: WO 2000/030609

(56) References cited:
- EP-A- 0 356 060
- EP-A- 0 377 091
- US-A- 5 422 116

## Description

The invention relates to the use of a chitosan salt for preparing an aqueous ophthalmic formulation for use as artificial tears having an antimicrobial activity.

Normal tears are a complex combination of substances which form three layers on the eye.

A very thin outer oily layer contains lipids from the meibomina glands in the eyelid, to reduce evaporation.

A thick middle watery layer, produced by the lachrymal glands, keeps the salinity and the acidity of the tears at proper level and also carries antibodies and other immune defense agents to defend the eye against infection.

A very thin inner mucus layer helps maintaining a stable tear film.

Dry eye syndrome, which is the decline of the quality or quantity of tears bathing the eye, can lead if untreated to scarring or ulceration of the cornea, and thus loss of vision.

In many cases, dry eye results from disorders of the various glands which work together to produce normal tears.

The dry eye syndrome may result of a very large range of causes such as aging, diseases and side effects of diseases, medications, contact lenses, environmental conditions, computer uses, and so one.

Artificial tears are the most common form of treatment for dry eye symptoms.

Artificial tears which are available at the present time contain generally as the major component glycerine, carboxymethyl cellulose, hydroxypropyl cellulose, hydroxypropyl methylcellulose, polyvinylalcohol or hyaluronic acid salts.

For example, EP-A-0,698,388 discloses an ophthalmic preparation for use as artificial tears containing hyaluronic acid salts as a viscosity thickener.

A frequent complication of dry eye is the appearance of infections due to the reduced quantity of lysozyme, one of the natural infection-fighting components present in the tears.

Another well known cause of surinfection is that unit-doses of artificial tears are often badly used by patients who prefer to keep the same dose for 2, 3 or more consecutive instillations than discard it as recommended, thus causing an infection or aggravating an existing infection.

Also known are problems of tolerance in relation with the quantity and/or the nature of active ingredients and/or preservatives contained in the artificial tears, because the treatment of dry eye with artificial tears is generally prescribed for a prolonged period which can be for life.

An object of the present invention is to provide an aqueous ophthalmic formulation for use as artificial tears which can be administrated to the eye in convenient drop form, and which prevents and cures infections or surinfections inherent to the treatment of dry eye by artificial tears without problems of tolerance even for a prolonged use.

According to the present invention, this object has been achieved as a result of the inexpected findings that an aqueous composition containing a very low concentration of chitosan salt can be useful as artificial tears having antimicrobial efficacy when administered in convenient drop form to the eye.

Chitosan is known as a chitin derivative obtained by partial to substantial deacetylation of chitin also named poly(N-acetyl-D-glucosamine), which is a naturally occuring biopolymer found in shellfish.

Chitosan contains free amine (-NH₂) groups and may be characterized as to the proportion of N-acetyl-D-glucosamine units and D-glucosamine units, and such is expressed as the degree of deacetylation of the fully acetylated polymer chitin.

Chitosan is known to have numerous pharmaceutical activities.

EP-A-0,356,060 discloses compositions for use in the treatment of wounds such as dermal ulcers, said compositions containing chitosan which show a combination of antimicrobial activity and wound healing capability. Under the conditions tested, bacteriostatic activity is reported to be observed beginning with a 0.5 % concentration of chitosan malate. However, bactericidal activity is reported to be obtained only with compositions containing 10 % or more of chitosan malate.

US-A-5,015,632 discloses that chitosan pyrithione has an antimicrobial activity against a number of strains tested, including Staphylococcus aureus, equivalent to that of sodium pyrithione but that chitosan acetate was not effective against the strains tested.

US-A-5,422,116 discloses that chitosan is useful for preparing a liquid ophthalmic aqueous sustained release delivery system which provides a slow and sustained release of the treating agents incorporated therein to the eye. A list of antibacterian agents which could be incorporated in the formulation as treating agent against infections is disclosed.

The present invention has for object the use of a chitosan salt having a deacetylation degree of 50 - 90 % and a molecular weight of 80,000 - 5,000,000 Da for the preparation of an aqueous ophthalmic formulation for use as artificial tears having antimicrobial activity, the chitosan salt being used in an amount of 0.05 - 3 wt/v % based on the total aqueous ophthalmic formulation, said aqueous ophthalmic formulation having a viscosity high enough to ensure an adequate precorneal residence time, and low enough to ensure that the formulation can easily be administered in drop form and a physiologically acceptable pH.

The present invention provides an aqueous ophthalmic formulation containing low concentration of chitosan salt for use as artificial tears having antimicrobial efficacy without further addition of an antimicrobial agent when administered in convenient drop form to the eye, which can prevent and cure infections or surinfections inherent to the treatment of dry eye by artificial tears even if unit doses are badly used as mentioned above, and which can be used without problems of tolerance even during a prolonged treatment such as for life.

An advantage of the use of chitosan under its salt form is that an addition of diluted acids, such as hydrochloric or acetic acids, to solubilize the chitosan having free amine groups is not required in the preparation of the ophthalmic formulation.

Other advantages of the present invention will appear in the following detailed description.

It is to be noted that in the present description and claims, the term "chitosan salt" means a chitosan containing ammonium (-NH₃⁺) groups with corresponding counterions (X⁻) instead of free amine (-NH₂) groups.

It is also to be noted that, as for chitosan, the "degree of deacetylation" in a sample of chitosan salt means the relative amounts of N-acetyl-D-glucosamine units and D-glucosammonium salt units present in the chitosan sample, and is expressed as the degree of deacetylation of the fully acetylated polymer chitin.

In the present invention, the term "antimicrobial activity" means both bactericidal and bacteriostatic activity.

The present invention will be now described in a more detailed manner.

Chitosan salt as used in the present invention is a chitosan salt having a counterion X⁻ derived from an inorganic or organic acid and having a deacetylation degree of 50 - 90 % and a weight average molecular weight of 80,000 - 5,000,000 Da.

Chitosan salt in various forms which can be used in the present invention is commercially available or can be prepared by a process based on deacetylation of chitin until the desired deacetylation degree, e.g. as described by Roberts, G.A.F., in "Chitin chemistry", Mc Millan Press LTD, Houndmills, p. 64-82 (1992), to obtain a chitosan including free amine groups, and addition of an organic or inorganic acid to the chitosan to convert the free amine (-NH₂) groups of chitosan into ammonium (-NH₃⁺) groups with corresponding counterions (X⁻), thus converting the chitosan into chitosan salt thereof, e.g. as described by Muzarelli, R.A., in "Natural chelating polymers", Pergamon Press, Oxford, p. 150-156 (1973). Commercial sources of chitosan salts are for example Pronova® Biopolymer, Drammen, Norway; Vanson Company, Redmond, Washington, USA; Nova Chem Limited, Armdale, Halifax, Nova Scotia, Canada; Biosynth A.G., Staad, Switzerland; Biopolymer Engineering, Inc., St-Paul, Minnesota, USA.

The weight average molecular weight of chitosan salt used in the present invention may be determined by size exclusion chromatography as mentioned hereafter.

Chitosan salts of molecular weights lower than 80,000 Da are not appropriate for use in the present invention because required viscosity may be not obtained.

Chitosan salts of molecular weights greater than 5,000,000 Da are not advantageous since they imply high manufacturing costs and also since they form very stiff gels which cannot be easily and reproducibly applied.

Preferably, the chitosan salt used in the present invention has a molecular weight of 160,000 - 580,000 Da.

The deacetylation degree of chitosan salts may be determined by a spectrophotometric method such as described in the literature by Muzarelli, R.A. and Ricchetti, R., in Carbohydr. Polym. 5, p. 461-472, 1985 or Muzarelli, R.A. and Richetti, R. in "Chitin in Nature and Technology", Plenum Press, p. 385-388, 1986.

Chitosan salt for use in the present invention has a deacetylation degree of 50 - 90% which means that the chitosan salt comprises 50 - 90 % of D-glucosammonium units with corresponding counterions and 50 - 10 % of N-acetyl-D-glycosamine units, respectively.

Preferably, chitosan salt as used in the present invention has a deacetylation degree of 83 % to 87 %.

For the purpose of the present invention, particularly preferred chitosan salts are chitosan hydrochloride and chitosan glutamate.

The aqueous ophthalmic formulation of the present invention comprises from 0.05 to 3 wt/v % of chitosan salt.

A formulation having a content of chitosan salt lower that 0.05 wt/v % is not appropriate for use as artificial tears having antimicrobial activity because the precorneal residence time becomes too short and the bactericidal effect becomes non-significant.

A formulation having a content of chitosan salt greater than 3 wt/v % is not appropriate for use as artificial tears because the presence of chitosan salt at this concentration may cause undesired side-effects such as irritation and intolerance.

Preferably, the chitosan salt is used in an amount of 0.5 to 1.5 wt/v %, based on the total aqueous ophthalmic formulation.

The aqueous ophthalmic formulation of the present invention has a viscosity of 10 - 500 mPa.s.

An ophthalmic formulation having a viscosity lower than 10 mPa.s is not advantageous for use as artificial tears because the precomeal residence time of the formulation becomes too short.

A formulation having a viscosity higher than 500 mPa.s is not appropriate for use as artificial tears because it forms a too stiff gel to be readily applied.

A particularly preferred ophthalmic formulation has a viscosity of 10 to 100 mPa.s.

For the purpose of the invention, the concentration of chitosan salt is adjusted according to the molecular weight and deacetylation degree of the chitosan salt used and according to the desired viscosity of the aqueous ophthalmic formulation.

It is to be noted that the aqueous ophthalmic formulation of the present invention has a Newtonian rheological behaviour.

According to the present invention, the aqueous ophthalmic formulation has a physiologically acceptable pH, preferably a pH of 5.4 - 7.0.

The osmolality of the aqueous ophthalmic formulation of the present invention ranges from 240 to 340 mosm/kg thus providing physiological acceptance.

The aqueous ophthalmic formulation of the present invention may be prepared according to conventional techniques by solubilizing the chitosan salt in the appropriate amount in a phosphate buffer solution (PBS) pH 7.4.

The aqueous ophthalmic formulation of the present invention can in particular be packaged as monodose units.

The aqueous ophthalmic formulation of the present invention has an antimicrobial activity against all bacteria which are sensitive to chitosan, in particular gram negative strains and gram positive strains, such as E.coli and S. aureus.

It is to be noted that the antimicrobial activity against S. aureus strains is particularly advantageous since it is known that such strains are very resistant and cause numerous problems in hospitals. It is to be noted that S. aureus is very often implicated in eye bacterial infections (conjunctivitis).

The aqueous ophthalmic formulation of the present invention has a very good wetting effect and a long precomeal residence time, and is very well tolerated. It is therefore appropriate to be used as artificial tears for prolonged treatment.

Due to its antimicrobial activity, the aqueous ophthalmic formulation of the present invention is particularly useful for preventing and treating infections when administered as artificial tears.

The aqueous ophthalmic formulation of the present invention is also advantageously used as artificial tears in the treatments of dry eye syndrome, eye irritations caused by environmental conditions or contact lenses, keratoconjunctivitis sicca, Sjögren's syndrome bacterial infections on the surface of the eye or related anterior structures caused by bacteria which are sensitive to chitosan, and in the prophylaxis of the bacterial infections in case of trauma and before or after surgery of the eye.

The aqueous ophthalmic formulation of the present invention can be used for topical administration to the eye in drop form.

To illustrate the antimicrobial activity of the formulations of the present invention used as artificial tears, tests will now be described with reference to an example wherein chitosan hydrochloride is being used as the chitosan salt.

The chitosan hydrochloride tested is named UPCI 110 and provided by Pronova®Biopolymer, Drammen, Norway.

The molecular weight of the UPCI 110 tested has been determined by size exclusion chromatography, with a Waters 600 E apparatus, combined with an autosampler (Waters TM717plus) and a Waters 410 differential refractometer. The conditions of analysis were the following :
- Column : series of 4 columns Ultrahydrogel® (7.8x300)
- Temperature : 30°C
- Flow rate : 0.8 ml/min
- Eluent : acetate buffer pH 4.2
0.1 % solution of UPCI 110 in acetate buffer was injected five times. By this method, the molecular weight of UPCI 110 has been determined to be 160,000 Da.

The deacetylation degree of UPCI 110 has been provided by the supplier and has been verified to be 87 % by the spectrophotometric method described in the above mentioned literature by Muzarelli, R.A. and Ricchetti, R.

The rheological evaluation of UPCI 110 has been tested at three increasing concentrations (0.5 %, 1.0 % and 1.5 %).

Rheological measurements have been made with a Bohlin Rheometer CS equipped with a system of control of the temperature (CS ETO). Data have been obtained under the following conditions :
- Temperature: 25°C
- Measuring system : Cone-plate 4/40 LS
- Shear stress : 5.97E-2 Pa
- Oscillation test

The results are shown in the following TABLE 1.

**TABLE 1**

| Concentration of UPCI 110 (%) | Viscosity¹ (mPa.s) |
|---|---|
| 0.5 | 10.0 |
| 1.0 | 17.4 |
| 1.5 | 30.7 |

| | |
|---|---|
| ¹Solution in a phosphate buffer solution (PBS) pH 7.4 | |

Tolerance of formulations containing UPCI 110 has been tested on rabbits and evaluated by using a confocal laser scanning ophthalmoscope (CLSO® Zeiss, Germany) modified by a set of lenses to examine the cornea instead of the retina. The rabbits were instillated with 25 µl of the test solution 4 times a day during 3 days. After the last instillation, the rabbits were sedated with an intramuscular injection of ketamine HCl/xylazine). Then, 25 µl of sodium fluorescein solution 0.5% in PBS were instilled in the eye to be tested. Fluorescein allows the injured areas to be selectively marked. The eye was then rinsed during 1 minute with a NaCl 0.9% solution. Then, the cornea was examined. Each aqueous ophthalmic solution was tested on 3 rabbits.

Corneal lesions after topical administration of ophthalmic solutions having different concentrations are expressed in percentage of corneal fluorescent areas.

The results are shown in the following TABLE 2.

**TABLE 2**

| Concentration of UPCI 110 (%) | Corneal lesions (%) |
|---|---|
| 0.5 | 6.8 |
| 1.0 | 10.2 |
| 1.5 | 12.3 |

Corneal lesions of less than 25 % are generally accepted as indicating a very good tolerance. The present results are therefore very satisfactory.

Clinical examination to evaluate discharge, corneal/conjunctival swelling or redness have confirmed CLSO results: formulations based on UPCI 110 were always very well tolerated.

Also, gamma scintigraphic studies on rabbits have shown that formulations of the present invention based on chitosan hydrochloride have a precorneal residence time longer than PBS or normal saline.

It is to be noted that the precorneal residence time can be increased by increasing the molecular weight or the concentration of the chitosan salt in the formulation, i.e. by increasing the viscosity of the formulation.

The evaluation of the antimicrobial efficacy of chitosan hydrochloride UPCI 110 has been tested against E. Coli strains which are representative of gram negative bacteria and against gram S. aureus strains which are representative of gram positive bacteria.

E. Coli strains used in the tests were isolated from clinical specimens in the University of Geneva.

S. aureus strains used in the tests were ATCC 25 925 obtained from the collection of Institut Pasteur (Paris, France).

The solutions tested and the conditions of test were as follows.

The solutions were prepared by serial dilutions from 300 µl to 10 µl of a solution containing 0.5% of chitosan hydrochloride of the type UPCI 110 in phosphate buffer solution (PBS) pH 7.4, completed ad 1.0 ml with the bacterial strains in suspension in a liquid medium (Brain-Heart-Infusion = BHI). Controls were phosphate buffer solution (PBS) pH 7.4 and artificial tear commercial solution (Protagent® Unit-dose), and the volumes used were the-same as the chitosan hydrochloride based solutions.

Each solution was incubated during 18 hours at 37°C.

After incubation, 50 µl of each solution were sprayed on solid plates (medium Mueller-Hinton 2) after appropriate dilution and left for incubation during 24 hours for bacterial counting. The number of bacteria suspended in BHI were counted before adding a solution.

The antimicrobial efficacy was determined twice for each bacterial strain.

The results of antimicrobial efficacy of UPCI 110 against E. Coli strains are shown in the following TABLE 3.

**TABLE 3**

| Solution tested | Volume (µl) | Concentration of UPCI 110 (wt/v %) | Number of bacteria |
|---|---|---|---|
| UPCI 110 | 300 | 0.15 | 0 |
| | 150 | 0.075 | 0 |
| | 75 | 0.0375 | 80,000 |
| | 30 | 0.015 | 1.8 x 10⁶ |
| | 10 | 0.005 | 2.8 x 10⁶ |
| PBS | 300 | 0 | 1.4 x 10¹⁰ |
| | 150 | 0 | 1.8 x 10¹⁰ |
| | 75 | 0 | 1.2 x 10¹⁰ |
| | 30 | 0 | 1.0 x 10¹⁰ |
| | 10 | 0 | 1.2 x 10⁹ |

The number of bacteria present in the liquid medium (BHI) at the beginning of the test was 80,000.

The results of antimicrobial efficacy of UPCI 110 against S. aureus strains are shown in the following TABLE 4.

**TABLE 4**

| Solution tested | Volume (µl) | Concentration of UPCI 110 (wt/v %) | Number of bacteria |
|---|---|---|---|
| UPCI 110 | 300 | 0.15 | 2,000 |
| | 150 | 0.075 | 3,000 |
| | 75 | 0.0375 | 22,000 |
| | 30 | 0.015 | 9.4 x 10⁵ |
| | 10 | 0.005 | 2.4 x 10⁹ |
| PBS | 300 | 0 | 4.3 x 10⁸ |
| | 150 | 0 | 8.8 x 10⁸ |
| | 75 | 0 | 4.7 x 10⁸ |
| | 30 | 0 | 3.8 x 10⁸ |
| | 10 | 0 | 1.1 x 10⁹ |
| Protagent SE ® | 300 | 0 | 8.4 x 10⁸ |
| | 150 | 0 | 7.0 x 10⁸ |
| | 75 | 0 | 2.2 x 10⁸ |
| | 30 | 0 | 6.8 x 10⁸ |
| | 10 | 0 | 2.1 x 10⁹ |

The number of bacteria present in the liquid medium(BHI) at the beginning of the test was 1.35 x 10⁶.

The above antimicrobial efficacy tests show the following.

In case of E. Coli, the bactericidal effect of the tested solution at concentrations of chitosan salt down to 0.0375 is clearly apparent, since in the presence of 0.0375% of chitosan salt, the number of bacteria (80,000) is the same as the number of bacteria suspended in BHI. At even lower concentrations, the solution still has a marked bacteriostatic effect, as shown by the controls.

In the case of S. aureus, the limit between the bactericidal and bacteriostatic effects is situated between concentrations of 0.005 and 0.015 % of chitosan.

In both cases, controls show that the volumes tested do not interfere with the nutritious medium (BHI), since bacteria can grow normally. Bacterial growth is similar in the commercial Protagent ® Unit-dose and in the phosphate buffer solution (PBS).

The following examples of aqueous ophthalmic formulations of the present invention are given for illustrative purposes and are not intended to limit the scope of the invention.

### EXAMPLES

### Example 1

2.5 g of chitosan hydrochloride UPCI 110 as referred above, having a molecular weight of 160,000 Da and a deacetylation degree of 87 %, is solubilized in 500 ml of a phosphate buffer solution (PBS) pH 7.4, at room temperature under magnetic stirring. The resulting aqueous ophthalmic formulation contains 0.5 % of chitosan hydrochloride, has a viscosity of 10 mPa.s, a pH of 5.47, and an osmolality of 290 mosm/kg.

### Example 2

7.5 g of chitosan hydrochloride UPCI 110 as referred above, having a molecular weight of 160,000 Da and a deacetylation degree of 87 %, is solubilized in 500 ml of a phosphate buffer solution (PBS) pH 7.4, at room temperature under magnetic stirring. The resulting aqueous ophthalmic formulation contains 1.5 % of chitosan hydrochloride, has a viscosity of 30.7 mPa.s, a pH of 5.48, and an osmolality of 290 mosm/kg.

### Example 3

5.0 g of chitosan glutamate UPG 210 obtained from Pronova®Biopolymer, Drammen, Norway, having a molecular weight of 580,000 Da and a deacetylation degree of 83 %, is solubilized in 500 ml of a phosphate buffer solution (PBS) pH 7.4, at room temperature under magnetic stirring. The resulting aqueous ophthalmic formulation contains 1.0 % of chitosan glutamate, has a viscosity of 54.5 mPa.s, a pH of 5.48 and an osmolality of 290 mosm/kg.

The formulations of Examples 1 and 2 may be packaged either in monodose units or in appropriate containers.

The formulations of Examples 1, 2 and 3 may be topically administered by instillation in the eye in convenient drop form.

## Claims

1. A use of a chitosan salt having a deacetylation degree of 50 - 90 % and a molecular weight of 80,000 - 5,000,000 Da for the preparation of an aqueous ophthalmic formulation for use as artificial tears having antimicrobial activity, the chitosan salt being used in an amount of 0.05 - 3 wt/v % based on the total aqueous ophthalmic formulation, said ophthalmic formulation having a viscosity high enough to ensure an adequate precorneal residence time, and low enough to ensure that the formulation can easily be administered in drop form and a physiologically acceptable pH.

2. The use according to claim 1, wherein said chitosan salt has a molecular weight of 160,000 - 580,000 Da.

3. The use according to claim 1 or 2, wherein said chitosan salt has a deacetylation degree of 83 - 87 %.

4. The use according to any one of claims 1 to 3, wherein the chitosan salt is chitosan hydrochloride or chitosan glutamate.

5. The use according to any one of claims 1 to 4, wherein said chitosan salt is used in an amount of 0.5 - 1.5 wt/v % based on the total aqueous ophthalmic formulation.

6. The use according to any one of claims 1 to 5, wherein said ophthalmic formulation has a viscosity of 10 - 100 mPa.s.

7. The use according to any one of claims 1 to 6, wherein said ophthalmic formulation has a pH of 5.4 - 7.0.

8. The use according to any one of claims 1 to 7, wherein said aqueous ophthalmic formulation is packaged as monodose units.

9. The use according to any one of claims 1 to 8, wherein the aqueous ophthalmic formulation has antimicrobial activity against bacteria which are sensitive to chitosan.

10. The use according to claim 9, wherein said bacteria are E.coli or S. aureus strains.

11. The use according to any one of claims 1 to 10, wherein said artificial tears are intended to treat dry eye syndrome.

12. The use according to any one of claims 1 to 10, wherein said artificial tears are intended to treat eye irritations caused by environmental conditions or contact lenses.

13. The use according to any one of claims 1 to 10, wherein said artificial tears are intended to treat keratoconjunctivitis sicca.

14. The use according to any one of claims 1 to 10, wherein said artificial tears are intended to treat Sjögren's syndrome.

15. The use according to any one of claims 1 to 10, wherein said artificial tears are intended to treat bacterial infections on the surface of the eye or related anterior structures caused by bacteria which are sensitive to chitosan.

16. The use according to any one of claims 1 to 10, wherein said artificial tears are intended for the prophylaxis of the bacterial infections in case of trauma and before or after surgery of the eye.

## Patentansprüche

1. Verwendung eines Chitosansalzes mit einem Deacetylierungsgrad von 50 - 90 % und einem Molekulargewicht von 80 000 - 5 000 000 Da für die Herstellung einer wässrigen opthalmischen Formulierung zur Verwendung als künstliche Tränen mit antimikrobieller Aktivität, wobei das Chitosansalz in einer Menge von 0,05 - 3 w/v-%, basierend auf der gesamten wässrigen opthalmischen Formulierung, verwendet wird, wobei die opthalmische Formulierung eine Viskosität, die ausreichend hoch ist, um eine angemessene präcorneale Verweilzeit sicherzustellen, und niedrig genug ist, um sicherzustellen, dass die Formulierung leicht in Tropfenform verabreicht werden kann, und einen physiologisch annehmbaren pH-Wert aufweist.

2. Verwendung nach Anspruch 1, wobei das Chitosansalz ein Molekulargewicht von 160 000 - 580 000Da aufweist.

3. Verwendung nach Anspruch 1 oder 2, wobei das Chitosansalz einen Deacetylierungsgrad von 83 - 87 % aufweist.

4. Verwendung nach mindestens einem der Ansprüche 1 bis 3, wobei das Chitosansalz Chitosanhydrochlorid oder Chitosanglutamat ist.

5. Verwendung nach mindestens einem der Ansprüche 1 bis 4, wobei das Chitosansalz in einer Menge von 0,5-1,5 w/v-%, basierend auf der gesamten wässrigen opthalmischen Formulierung, verwendet wird.

6. Verwendung nach mindestens einem der Ansprüche 1 bis 5, wobei die opthalmische Formulierung eine Viskosität von 10-100 mPa.s besitzt.

7. Verwendung nach mindestens einem der Ansprüche 1 bis 6, wobei die opthalmische Formulierung einen pH-Wert von 5,4-7,0 besitzt.

8. Verwendung nach mindestens einem der Ansprüche 1 bis 7, wobei die wässrige opthalmische Formulierung als Einzeldosiseinheiten verpackt ist.

9. Verwendung nach mindestens einem der Ansprüche 1 bis 8, wobei die wässrige opthalmische Formulierung antimikrobielle Aktivität gegen Bakterien aufweist, welche gegen Chitosan empfindlich sind.

10. Verwendung nach Anspruch 9, wobei es sich bei den Bakterien um E.colioder S.aureus-Stämme handelt.

11. Verwendung nach mindestens einem der Ansprüche 1 bis 10, wobei die künstlichen Tränen zur Behandlung des Trockene-Augen-Syndroms beabsichtigt sind.

12. Verwendung nach mindestens einem der Ansprüche 1 bis 10, wobei die künstlichen Tränen zur Behandlung von Augenreizungen beabsichtigt sind, welche durch Umwelteinflüsse oder Kontaktlinsen hervorgerufen werden.

13. Verwendung nach mindestens einem der Ansprüche 1 bis 10, wobei die künstlichen Tränen zur Behandlung von Keratoconjunctivitis sicca beabsichtigt sind.

14. Verwendung nach mindestens einem der Ansprüche 1 bis 10, wobei die künstlichen Tränen zur Behandlung von Sjögren-Syndrom beabsichtigt sind

15. Verwendung nach mindestens einem der Ansprüche 1 bis 10, wobei die künstlichen Tränen zur Behandlung von bakteriellen Infektionen auf der Oberfläche des Auges oder verwandten anterioren Strukturen, verursacht von Bakterien, welche gegen Chitosan empfindlich sind, beabsichtigt sind.

16. Verwendung nach mindestens einem der Ansprüche 1 bis 10, wobei die künstlichen Tränen für die Prophylaxe der bakteriellen Infektionen im Falle einer Verletzung und vor oder nach einem chirurgischen Eingriff am Auge beabsichtigt sind.

## Revendications

1. Utilisation d'un sel de chitosan ayant un degré de désacétylation de 50 - 90 % et un poids moléculaire de 80 000 - 5 000 000 Da pour la préparation d'une formulation ophtalmique aqueuse employée en tant que larmes artificielles ayant une activité antimicrobienne, le sel de chitosan étant utilisé à raison de 0,05 - 3 % poids / volume sur la base de la totalité de la formulation ophtalmique aqueuse, ladite formulation ophtalmique ayant une viscosité suffisamment élevée pour assurer un temps de résidence adéquat sur la cornée et suffisamment bas pour permettre une administration facile de la formulation sous la forme de collyre, et un pH physiologiquement acceptable.

2. Utilisation selon la revendication 1, où ledit sel de chitosan a un poids moléculaire de 160 000 - 580 000 Da.

3. Utilisation selon la revendication 1 ou la revendication 2, où ledit sel de chitosan a un degré de désacétylation de 83 - 87 %.

4. Utilisation selon une quelconque des revendications 1 à 3, où le sel de chitosan est le chlorhydrate de chitosan ou le glutamate de chitosan.

5. Utilisation selon une quelconque des revendications 1 à 4, où ledit sel de chitosan est utilisé à raison de 0,5 - 1,5 % en poids / volume, sur la base de la totalité de la formulation ophtalmique aqueuse.

6. Utilisation selon une quelconque des revendications 1 à 5, où ladite formulation ophtalmique a une viscosité de 10 - 100 mPa.s.

7. Utilisation selon une quelconque des revendications 1 à 6, où ladite formulation ophtalmique a un pH de 5,4 - 7,0.

8. Utilisation selon une quelconque des revendications 1 à 7, où ladite formulation ophtalmique aqueuse est conditionnée dans des unités à dose unique.

9. Utilisation selon une quelconque des revendications 1 à 8, où la formulation ophtalmique aqueuse a une activité antimicrobienne contre des bactéries qui sont sensibles vis-à-vis du chitosan.

10. Utilisation selon la revendication 9, où lesdites bactéries sont des souches d'E. coli ou de S. aureus.

11. Utilisation selon une quelconque des revendications 1 à 10, où lesdites larmes artificielles sont destinées à traiter le syndrome de l'oeil sec.

12. Utilisation selon une quelconque des revendications 1 à 10, où lesdites larmes artificielles sont destinées au traitement d'irritations oculaires, provoquées par les conditions environnementales ou par le port de lentilles de contact.

13. Utilisation selon une quelconque des revendications 1 à 10, où lesdites larmes artificielles sont destinées au traitement de la kératoconjonctivité sèche.

14. Utilisation selon une quelconque des revendications 1 à 10, où lesdites larmes artificielles sont destinées au traitement du syndrome de Sjogren.

15. Utilisation selon une quelconque des revendications 1 à 10, où lesdites larmes artificielles sont destinées au traitement d'infections bactériennes sur la surface oculaire ou sur des structures antérieures annexes, provoquées par des bactéries qui sont sensibles au chitosan.

16. Utilisation selon une quelconque des revendications 1 à 10, où lesdites larmes artificielles sont destinées au traitement prophylactique d'infections bactériennes dans le cas de traumatismes et avant ou après une opération chirurgicale effectuée sur l'oeil.
